# EUROPÄISCHE PATENTSCHRIFT

(11) **EP 1 032 325 B1**
(45) Veröffentlichungstag und Bekanntmachung des Hinweises auf die Patenterteilung: **01.09.2004**
(21) Anmeldenummer: 98922659.2
(22) Anmeldetag: 07.04.1998
(51) Int. Cl.: A61C 19/04, A61C 3/03

(54) **DIAGNOSEGERÄT ZUR BESTIMMUNG DER REMINERALISIERBARKEIT VON HARTGEWEBE**
DIAGNOSTIC APPARATUS FOR DETERMINING THE REMINERALIZATION ABILITY OF TISSUE
APPAREIL DE DIAGNOSTIC POUR DETERMINER L'APTITUDE A LA REMINERALISATION DE TISSU DUR

(30) Priorität: 07.04.1997 DE 19714167
(43) Veröffentlichungstag der Anmeldung: 06.09.2000
(73) Patentinhaber: Hahn, Rainer, Dr., 72074 Tübingen (DE)
(72) Erfinder: Hahn, Rainer, Dr., 72074 Tübingen (DE)
(74) Vertreter: Ostertag, Reinhard
(86) Internationale Anmeldenummer: PCT/EP1998/002017
(87) Internationale Veröffentlichungsnummer: WO 1998/044868

(56) Entgegenhaltungen:
- WO-A-95/04506
- WO-A-95/22938
- US-A- 4 215 698
- DATABASE WPI Section Ch, Week 9204 Derwent Publications Ltd., London, GB; Class B04, AN 92-031985 XP002076594 & SU 1 649 429 A (ODESS MEDICAL INST) , 15. Mai 1991
- DATABASE WPI Section Ch, Week 8936 Derwent Publications Ltd., London, GB; Class D21, AN 89-261605 XP002076595 & SU 1 439 507 A (OMSK MED INST) , 23. November 1988

## Beschreibung

Die Erfindung betrifft ein Diagnosegerät, welches die Fähigkeit eines Hartgewebes zur Remineralisierung messen kann.

Soweit Hartgewebe (im Nachfolgenden wird teilweise stellvertretend auch von Zahngewebe gesprochen) defekte Bereiche (Läsionen) aufweist, die an der Gewebeoberfläche liegen, läßt sich eine Diagnose in der Regel visuell und taktil (Kratzen mit Spitze) treffen.

Die US 4 215 698 offenbart ein Diagnosegerät für Karies, welches die elektrischen Zahngewebeeigenschaften, besonders die Wechselstromleitfähigkeit mißt. Sie beschreibt hierfür eine Testsonde, die auf den zu untersuchenden Zahn aufgesetzt wird. Die Leitfähigkeitsergebnisse lassen Rückschlüsse auf das Maß an Kariesbefall eines untersuchten Zahnes zu; Aussagen über die Lage der kariösen Stelle im Zahnvolumen sind nicht ableitbar.

Nicht an der Oberfläche gelegene Gewebeläsuren lassen sich nur anhaltsweise durch Röntgenaufnahmen ermitteln. Da letztere nur allgemein die Absorption von Röntgenstrahlen wiedergeben, dabei aber offen bleibt, welche Anteile der Absorption auf schon in gesundem Zustand vorhandene unterschiedliche Gewebekonsistenz und Zahngeometrie zurückzuführen ist, läßt sich die Schwere und die Ausdehnung derartiger Läsionen nur schwer abschätzen. In der Regel ist es daher notwendig, einen größeren Zugang zu derartigen versteckten Läsionen zu schaffen, über welchen dann die klassische Versorgung der Läsion erfolgt, nämlich ein Abtragen alles kranken Gewebes und ein Wiederfüllen der so erzeugten Kavität mit plastischem Füllmaterial und/oder einem Restaurationsteil.

Bei diesem Vorgehen müssen oft erhebliche Volumina an gesundem Gewebe entfernt werden. Dabei ist eine Teilmenge der so geöffneten Läsionen nicht irreversibel geschädigt, könnte vielmehr durch Remineralisierung geheilt werden.

Die vorliegende Erfindung beschäftigt sich daher allgemein mit dem Problemkreis, unter weitestgehender Erhaltung gesunden Gewebes in der Nachbarschaft einer Läsion festzustellen, inwieweit die Gewebeläsion durch Remineralisierung therapierbar ist, damit gebenenfalls eine solche Therapie durchgeführt werden kann.

Hierzu schafft die Erfindung ein Gerät zur Bestimmung der Remineralisierungsfähigkeit von Hartgewebe, insbesondere kariösem Zahngewebe, welches die im Anspruch 1 angegebenen Merkmale aufweist,

Dabei wird die Gewebeeigenschaft der Porosität als Kriterium für die Remineralisierbarkeit herangezogen. Die Porosität ihrerseits kann mittels des Durchtritts eines Diagnosfluids festgestellt werden.

Dabei erlaubt das in Anspruch 1 angegebene Gerät ein paßgenaues Einführen eines Sondenabschnittes eines Meßkopfes in einen im Hartgewebe erzeugten Arbeitskanal, so daß schon die einander gegenüberliegenden Oberflächen von Arbeitskanal und Sondenabschnitt eine Spaltdichtung bilden, deren Dichtwirkung durch ein vom Meßkopf getragenes Dichtelement verstärkt wird.

Das im Anspruch 1 angegebene Diagnosegerät mißt die Gewebeeigenschaft der Porosität des Hartgewebes bzw. des im Sinne eines Karies veränderten Hartgewebes. Dabei werden Unterschiede zu den entsprechenden Eigenschaften des gesunden Gewebes als Maß für die Stärke der Läsion genommen. Unter Gewebeeigenschaften sollen in den Ansprüchen und der vorliegenden Beschreibung sowohl volumeneigenschaften als auch Oberflächeneigenschaften verstanden werden.

Die zur Ermittlung der Porosität in erster Linie verwendete Meßmethode ist die Ermittlung der Durchlässigkeit des Gewebes für ein Diagnosefluid, vorzugsweise eine Flüssigkeit, insbesondere eine wässrige Lösung. Je besser die Qualität der durch das Gewebe gebildeten Diffusionsbarriere ist, um so weniger Gewebeschäden liegen vor. Die Durchlässigkeit von Hartgewebe für wässrige Lösungen ist ein direktes Maß für eine Remineralisierbarkeit des Gewebes, denn genau auf demjenigen Wege, der auch zur Messung verwendet wird, erfolgt auch die Remineralisierung.

Mit einem erfindungsgemäßen Diagnosegerät läßt sich die Ausdehnung und die Stärke der Schädigung eines Gewebes besonders schonend durch fluidische Messung seiner Porosität bestimmen. Wie oben schon dargelegt, ist die Fähigkeit von Gewebe, eine Diffusionsbarriere für Fluids zu bilden, ein Maß für die Gesundheit von Gewebe überhaupt. Hier ist zu berücksichtigen, daß an einer kariösen Erkrankung von Zähnen oftmals das Dentin mit seiner Kanälchen-Struktur und hohem Wassergehalt, kariöser Schmelz mit seinem im Vergleich zu gesundem Schmelz höheren relativen Anteilen an Wasser und organischem Gewebe und kariös verändertes Dentin mit seinen im Vergleich zu gesundem Dentin ebenfalls höheren relativen Anteilen an Wasser und organischem Gewebe oftmals gleichermaßen beteiligt sind. Eine Durchlässigkeitsmessung, die an einem speziellen erkrankten Gewebebereich durchgeführt wird, kann zwar nicht im einzelnen auflösen, welche Gewebeart in welchem Umfange geschädigt ist, sie gibt aber insgesamt eine gute Information über die Ausdehnung und Stärke der Schädigung.

Die Remineralisierung ist möglich bei kariösen Volumina, bei denen die Zahnstruktur bzw. Dentinstruktur und Schmelzstruktur des Kariesvolumens noch weitgehend intakt ist, jedoch bezüglich ihres Mineralgehaltes gegenüber vergleichbarem "gesundem" Zahnhartgewebe reduziert ist. Die Mineralien für eine Remineralisierung stammen bei natürlicher Remineralisierung aus dem Speichel. Die Remineralisierung führt zurück zu weitgehend als gesund anzusehendem Hartgewebe (restitutio ad integrum).

Bei stärker geschädigten Hartgeweben ist aufgrund struktureller Einbrüche (meistens lokalisiert) eine Remineralisierung nicht mehr möglich. Zur Behandlung müssen derartige kariöse Volumina (ggf. zusätzlich benachbarte an sich remineralisationsfähige Gewebevolumina) nach vorausgehender Konditionierung oder Ätzung mit einem künstlichen Material gefüllt werden, welches die mechanische Festigkeit wiederherstellt und ein Hindernis für die Weiterentwicklung der Karies ist. Dieses Füllen der strukturellen Einbrüche erfolgt dadurch, daß man einen Kunststoff, ein Polymer oder Polymerkomposit in die nicht mehr remineralsierbare kranke Zahnstruktur infiltriert. Da dieses künstliche Material in gesunder Zahnstruktur enthaltene Gewebekomponenten ersetzt, spricht man von Reinfiltration.

Vorteilhafte Weiterbildungen der Erfindung sind in Unteransprüchen angegeben.

Die Weiterbildung der Erfindung gemäß Anspruch 2 erlaubt es, Intensität und Ausdehnung eines kranken Gewebebereiches zu bestimmen.

Ein Sondenstellungsgeber, wie er im Anspruch 3 angegeben ist, zeichnet sich durch mechanisch besonders einfachen Aufbau aus.

Die Weiterbildung der Erfindung gemäß Anspruch 4 gestattet es, auf einfache Weise Grundwerte der Porosität zu bestimmen, wie sie bei dem betrachteten Patienten in der Nachbarschaft der Läsion angefunden werden. Unter Bezugnahme auf diese individuellen und ortsspezifischen Referenzwerte lassen sich krankhafte Abweichungen dann besonders fein ermitteln.

Die Weiterbildung der Erfindung gemäß Anspruch 5 erlaubt es, in einem in dem Gewebe erzeugten Arbeitskanal die Porosität nicht nur in axialer Richtung sondern auch in radialer Richtung aufgelöst zu messen.

Die Weiterbildung der Erfindung gemäß Anspruch 6 ist im Hinblick auf eine für den Zahnarzt ergonomisch günstige Handhabung des Diagnosegerätes in unterschiedlichen beengten Bereichen des Mundes eines Patienten von Vorteil.

Auch die Weiterbildung der Erfindung gemäß Anspruch 7 dient der Ergonomie des Diagnosegerätes.

Die Weiterbildung der Erfindung gemäß Anspruch 8 erlaubt ein unbehindertes Drehen des Greifteiles um seine Längsachse.

Die Weiterbildung der Erfindung gemäß Anspruch 9 erlaubt ein Untersuchen der Porosität des Gewebes in weiten Grenzen.

Ein Gerät gemäß Anspruch 10 läßt sich besonders einfach handhaben.

Dabei wird mit der Weiterbildung der Erfindung gemäß den Ansprüchen 11 und 12 erreicht, daß durch die Diagnosezwecken dienende Messung keine zusätzlichen nennenswerten Defekte im Gewebe erzeugt werden.

Verwendet man als Meßfluid ein Gas, wie im Anspruch 14 angegeben, so können auch kleine Gewebedefekte gut bestimmt werden, und in der Regel sind kleine Druckabweichungen vom Normaldruck zur Diagnose ausreichend.

Die Weiterbildung der Erfindung gemäß Anspruch 15 gestattet dabei eine Differenzierung stärker geschädigter Gewebebereiche, da mehr Fluid für einen Meßvorgang zur Verfügung steht.

Die Weiterbildung der Erfindung gemäß Anspruch 16 ist im Hinblick auf das quantitative Ausmessen stärkerer Gewebedefekte von Vorteil.

Mit einem Diagnosegerät gemäß Anspruch 17 lassen sich die Leckströme durch defekte Gewebebereiche sehr einfach visuell erkennen und messen.

Ein Diagnosegerät gemäß Anspruch 18 baut besonders kompakt und ist mechanisch besonders einfach, da die Feder des Stellungsgebers zugleich als Vorspannfeder für das Dichtelement dienen kann.

Ein Diagnosegerät gemäß Anspruch 19 kann die Gewebeeigenschaften mit axialer und radialer Auflösung in den einen Arbeitskanal umgebenden Gewebebereichen ermitteln.

Die Weiterbildung der Erfindung gemäß Anspruch 20 ist im Hinblick auf eine besonders gute Abdichtung des Arbeitskanales gegen die Umgebung von Vorteil, da zwischen der Außenfläche des Sondenabschnittes und der Innenfläche des schlauchförmigen Dichtungselementes, insbesondere aber auch zwischen dem aufgekelchten Ende des Dichtelementes und dem Dichtbund des Sondenabschnittes, eine ausgedehnte gute Dichtfläche besteht.

Die Weiterbildung der Erfindung gemäß Anspruch 21 mißt den Fluidaustausch zwischen Gewebe und Meßsonde. Auf diese Weise kann der Zustand von Geweben ermittelt werden, die weiter im Inneren eines Gewebes liegen, ebenso bei Läsionen, die noch nicht zu Fluid-Leckagen zur Oberfläche des Gewebes geführt haben.

Überdruckwerte für das Diagnosegerät, wie sie im Anspruch 23 angegeben sind, erlauben einen besonders aussagekräftigen Rückschluß auf Art und Ausmaß einer Hartgewebeläsion.

Die Weiterbildung der Erfindung gemäß Anspruch 24 ist im Hinblick auf eine vorübergehende Erniedrigung der Viskosität des Diagnose- oder Behandlungsmediums und damit im Hinblick auf ein gutes Zuführen dieser Medien zur Diagnose- oder Behandlungsstelle von Vorteil.

Bei einem Gerät gemäß Anspruch 25 erfolgt diese Viskositätsherabsetzung direkt bei dem nur dünnen Zuführteil.

Die Weiterbildung der Erfindung gemäß Anspruch 26 ermöglicht es, auch das Abziehen verbrauchten Mediums durch das Zuführteil unter vorübergehender Herabsetzung seiner Viskosität zu besorgen.

Die Unterschiede zwischen gesundem und krankem Gewebe kann man in vielen Fällen dadurch verstärken, daß man über das Diagnosegerät Diagnosefluid mit einer Markiersubstanz zuführt, die von kranken Gewebebereichen anders (stärker oder schwächer) auf- oder angenommen wird wie von gesunden Gewebebereichen. Diese Markiersubstanzen sind im Hinblick auf die jeweils verwendete Meßmethode gewählt, wie im Anspruch 27 angegeben.

Die Weiterbildung der Erfindung gemäß Anspruch 28 ermöglicht ein verbessertes optisches Erkennen kranker Gewebebereiche.

Die im Anspruch 29 angegebenen partikelförmigen Komponenten des Diagnosefluids dienen speziellen diagnostischen Zwecken, z.B. zur röntgenologischen Markierung eines Defektvolumens. Zugleich können sie Fülllpartikel für Defektvolumina darstellen.

Mit einem Diagnosemedium gemäß Anspruch 30 kann man die Porösität stark geschädigter Gewebebereiche durch sukzessives Zusetzen der großen Poren messen.

Dabei haben sich die im Anspruch 31 angegebenen Partikelgrößen in der Praxis besonders gut bewährt.

Die Weiterbildung der Erfindung gemäß Anspruch 32 erlaubt ein kontinuierliches Einstellen der Viskosität des Diagnosefluids und damit an Meßbereiche unterschiedlicher Schädigungsklassen.

Die Weiterbildungen der Erfindung gemäß den Ansprüchen 33 und 34 erlauben eine Messung der Porosität von Gewebe unter gleichzeitigem Entfernen von Gewebeanteilen, die zur Neubildung gesunden Gewebes nicht mehr beitragen können.

Nachstehend wird die Erfindung anhand von Ausführungsbeispielen unter Bezugnahme auf die beiliegende Zeichnung näher erläutert. In dieser zeigen:
- Figur 1:: eine perspektivische Ansicht eines Backenzahnes mit einem kranken Gewebebereich, der dem Nachbarzahn benachbart ist;
- Figur 2:: einen vertikalen Schnitt durch den in Figur 1 gezeigten Backenzahn längs der dortigen Schnittlinie II-II;
- Figur 3:: einen horizontalen Teilschnitt durch den in Figur 1 gezeigten Backenzahn längs der dortigen Schnittlinie III-III;
- Figur 4:: eine schematische Darstellung eines Diagnoseund Behandlungsgerätes für im Inneren eines Zahnes liegendes kariöses Gewebe sowie einer zugehörigen Fluid-Versorgungseinheit und einer zugehörigen elektronischen Auswerteund Steuereinheit;
- Figur 5:: eine seitliche, teilweise geschnittene Ansicht einer abgewandelten Dichtstelle zwischen einem Arbteitsabschnitt eines Diagnoselungsgerätes und einem in einem Zahn erzeugten Arbeitskanal;
- Figuren 6-9:: Diagramme, in welchen veranschaulicht ist, wie sich der Druck in zu unterschiedlich stark beschädigten Zahnbereichen führenden Arbeitskanälen bei Verwendung eines Diagnosegerätes gemäß Figur 4 in Abhängigkeit von der Zeit ändert;
- Figur 10:: eine zu Figur 4 ähnliche Darstellung eines kombinierten Bohr-, Diagnose- und Behandlungsgerätes; und
- Figur 11:: eine schematische Darstellung einer Absaugeinrichtung für überschüssiges Diagnosefluid.

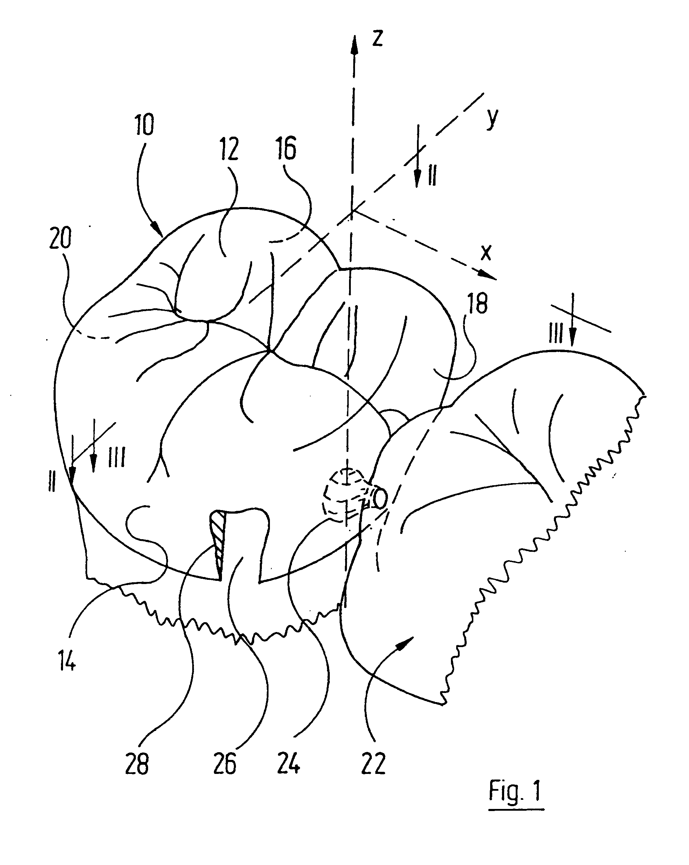

In Figur 1 ist mit 10 insgesamt ein Backenzahn bezeichnet.

Dieser hat eine obenliegende Kaufläche 12 (okklusale Fläche), eine wangenseitige, äußere oder bukkale Begrenzungsfläche 14, eine innenliegende oder orale Begrenzungsfläche 16, eine zum vorderen Kieferende weisende vordere oder approximal-mesiale Begrenzungsfläche 18 und eine hintere oder approximal-distale Begrenzungsfläche 20.

Ein benachbarter Backenzahn ist bei 22 gezeigt.

In dem den Backenzahn 22 benachbarten Bereich hat der Backenzahn 10 einen kranken Gewebebereich (Läsion) 24. Für die Zwecke der Beschreibung ist der Einfachheit halber angenommen, daß die Läsion im wesentlichen die Form eines Rotationsellypsoides hat, wobei sich versteht, daß in der Praxis Läsionen zum einen in der Regel unsymmetrische Gebilde sind und darüber hinaus keine scharfen Begrenzungsflächen haben, der Übergang zwischen krankem und gesundem Gewebe fließend ist. Ferner sei angenommen, daß die Läsion 24 nicht nur die in Figur 1 mit 28 bezeichnete Zahnschmelz-Kappe betrifft, sondern sich auch in das Dentinvolumen 26 des Backenzahnes 10 hinein erstreckt. Die Außenfläche der Zahnschmelzkappe 28 zeigt jedoch noch keine wesentlichen strukturellen Einbrüche.

Die in Figur 1 dargestellte Lage und Abmessung der Läsion 24 ist grob aus einer vom Backenzahn 10 gemachten Röntgenaufnahme abgeleitet. Auf Grund des Röntgenbildes kann nicht entschieden werden, ob es sich bei der Läsion 24 um einen so stark geschädigten Gewebebereich handelt, daß strukturelle Einbrüche, Porositäten oder gar eine Kavität vorliegt, welche reinfiltriert oder gar nach Kariesentfernung konventionell mit einer Füllung versorgt werden müssen. Es könnte sich bei der Läsion 24 auch nur um einen anfänglich geschädigten Gewebebereich handeln, der durch Remineralisierung wieder in gesunden Zustand zurückgebracht werden kann.

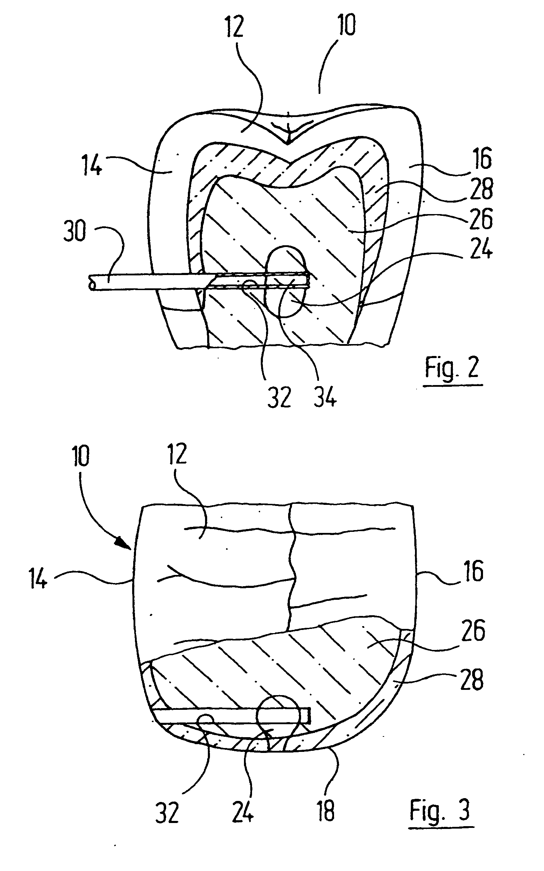

Um Information über den Krankheitszustand des Gewebebereichs 24 zu bekommen, wird von der bukkalen Begrenzungsfläche 14 her unter Verwendung eines hohlen Kernbohrers 30 (vgl. Figur 2) durch die Zahnschmelzkappe 28 und das Dentinvolumen 26 ein Arbeitskanal 32 so weit eingebohrt, daß sein Ende gemäß der aus den Röntgenbildern entnommenen Rohinformation jenseits des kranken Gewebebereiches 24 wieder in gesundem Dentin liegt, wie in Figur 2 dargestellt.

Der Durchmesser des Arbeitskanales 32 kann von etwa 0,6 mm bis 0,8 mm betragen.

Für eine genauere Auflösung der Größe und Stärke der Läsion kann wie folgt vorgegangen werden:

Es wird schrittweise auf das Zentrum der Läsion zugebohrt und sukzessive werden an mehreren Stellen vom gesunden Gewebe kommend ins kranke hineinerstreckend Messungen durchgeführt, vorzugsweise durch Bestimmung von Leckströmen, welche von der Bohrung über krankes Gewebe zur Zahnoberfläche verlaufen ("natürlicher Karieszugang").

Der Bohrkern bzw. dessen Analyse gibt die Sicherheit, daß man nicht vollständig an der betroffenen Läsion vorbeigebohrt hat. Die Analyse des Bohrkernes ist ein optionaler und nicht unverzichtbarer Schritt. Ergibt sich aufgrund der Leckstrommessung, daß die Gewebestruktur noch nicht stärker beeinträchtigt ist, so kann die Läsion durch Remineralisierung geheilt werden.

Ist der Zugang (natürlicher Karieszugang) strukturell so weit geschädigt, daß eine Remineralisation nicht mehr möglich ist, wird plötzlich bei einem Schritt des stufenweisen Bohrens des Arbeitskanals und jeweils anschließenden Messung eine Leckage feststellbar sein.

Der durch Remineralisieren nicht mehr heilbare Defekt kann dann zur Entfernung stark geschädigter Gewebeteile spanend bearbeitet werden. Dies kann unter Verwendung von Ultraschall-Bearbeitungswerkzeugen oder mechanischen Werkzeugen ggf. unter Unterstützung durch in Flüssigkeit suspendierte abrasive Partikel erfolgen. Nach Defektkonditionierung kann dann eine Reinfiltration von flüssigem und später aushärtendem Material erfolgen.

Beim Herausziehen des Kernbohrers 30 aus dem Backenzahn 10 wird ein von diesem umschlossene Bohrkern 34 mit herausgezogen. Durch ein geeignetes Werkzeug, z.B. einen dünnen Draht, kann man den Bohrkern 34 aus dem hohlen Kernbohrer 30 axial herausstoßen, und durch Betrachten (gegebenenfalls unter Anfärben, z.B. mit Fuchsin) können die kranken Gewebebereiche von den gesunden Gewebebereichen rasch unterschieden werden. Gegebenenfalls können chemische und/oder biologische Analysen der verschiedenen Abschnitte des Bohrkernes 34 zusätzlich durchgeführt werden.

Figur 3 zeigt den durch den Kernbohrer 30 erzeugten nahe bei der approximalen Begrenzungsfläche des Backenzahnes 10 verlaufenden Arbeitskanal 32.

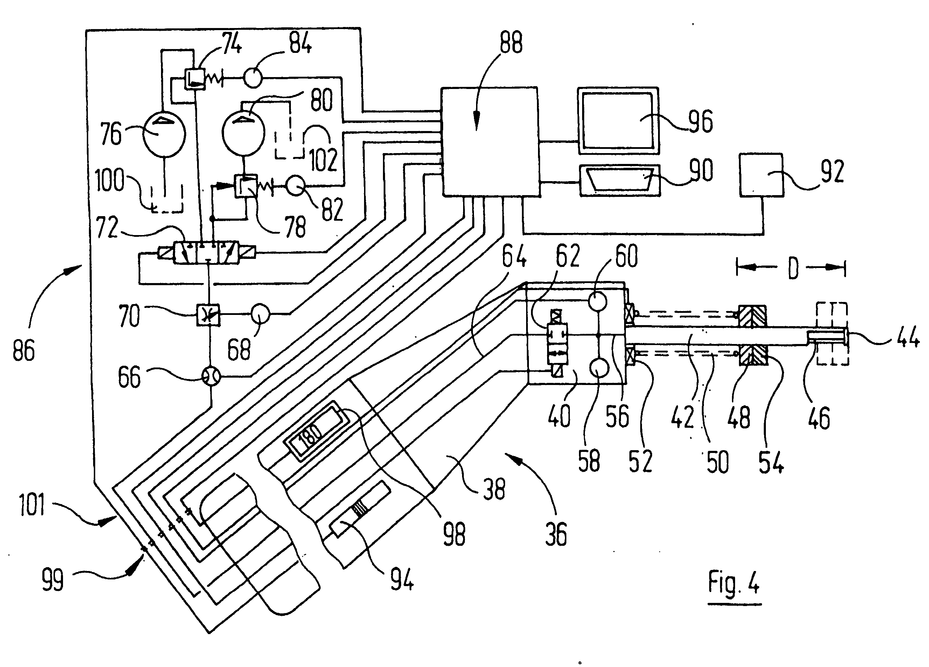

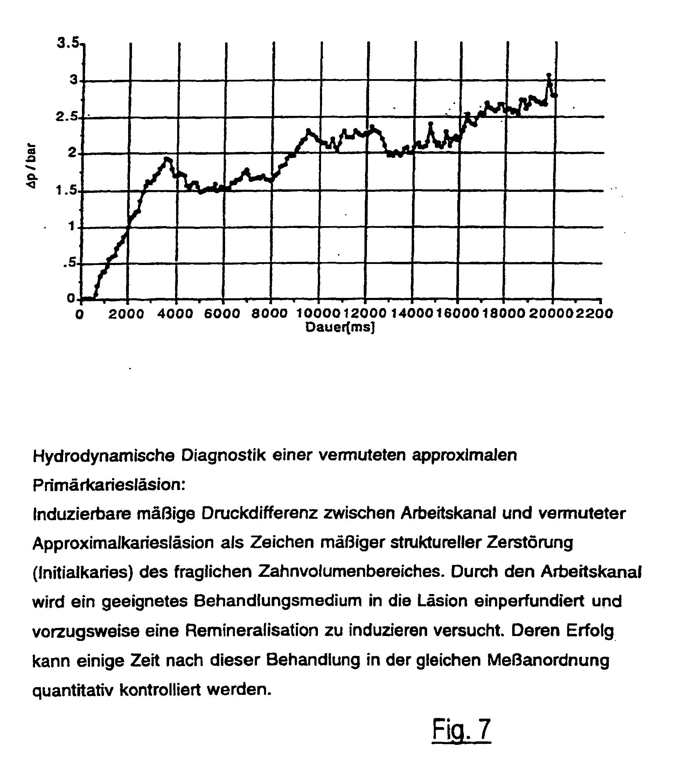

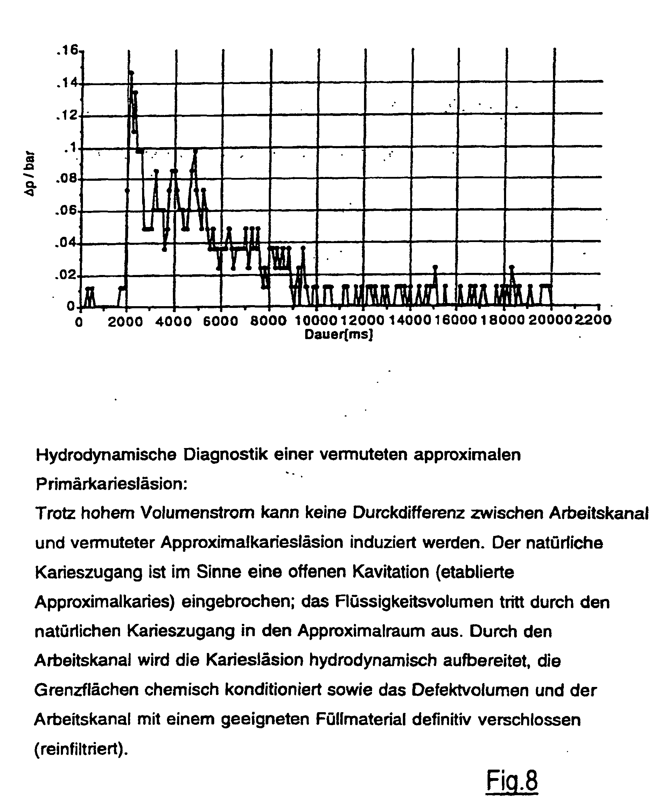

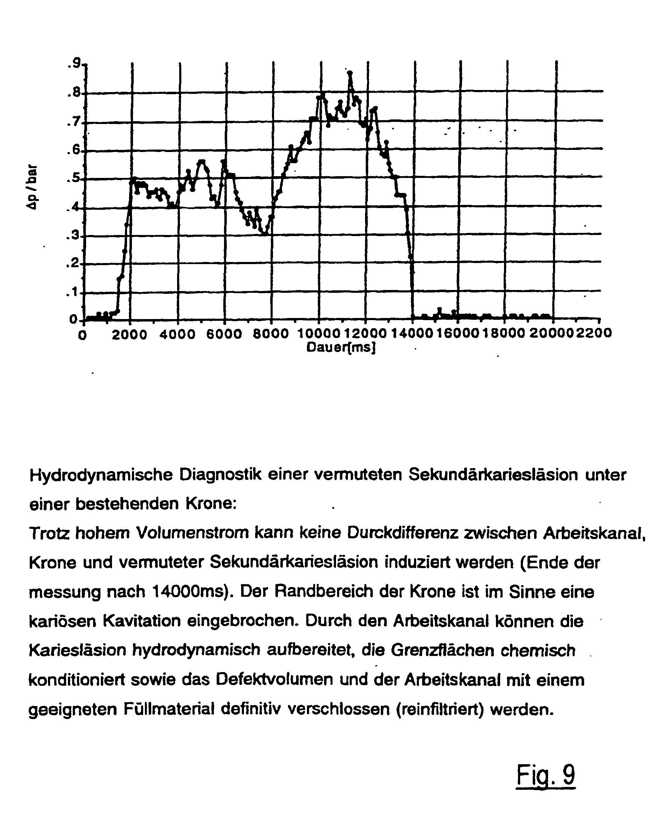

Der Arbeitskanal 32 kann zunächst dazu dienen, ein Diagnosegerät ins Innere des Zahnes einzuführen, mit welchem nicht nur gesunde und kranke Gewebebereiche unterschieden werden können, sondern auch erkannt werden kann, ob kranke Gewebebereiche durch Remineralisierung geheilt werden können. Derartige Diagnosegeräte werden zunächst nun unter Bezugnahme auf die Figuren 4-11 beschrieben, wobei das Gerät nach Figur 4 zugleich auch ein Behandlungsgerät ist.

Der Arbeitskanal 32 kann ferner zum Einführen oder Ansetzen von Behandlungsgeräten dienen, wie sie weiter unten unter Bezugnahme auf die Figuren 12-15 erläutert werden, wozu noch das kombinierte Diagnose- und Behandlungsgerät nach Figur 4 kommt.

In Figur 4 ist ein Diagnose- und Behandlungshandstück für kariöses Zahngewebe insgesamt mit 36 bezeichnet.

Das Handstück 36 hat ein Griffteil 38, das ähnliche axiale und radiale Abmessung aufweist wie ein Bohrerhandstück. Am Griffteil 38 ist ein insgesamt mit 40 bezeichneter Arbeitskopf angebracht. Dieser trägt einen hohlen zylindrischen Arbeitsabschnitt 42. Letzterer hat an seinem Ende eine Anschlagplatte 44, die über drei gleich in Umfangsrichtung verteilte dünne Distanzstäbe 46 mit dem offenen Ende des zylindrischen Arbeitsabschnittes 42 verbunden ist. Auf diese Weise ist gewährleistet, daß der Arbeitsabschnitt 42 nicht so weit in den Arbeitskanal 32 eingeführt werden kann, daß sein offenes Ende durch den Boden des Arbeitskanales 32 verschlossen wird. Die Anschlagplatte 44 bildet im Arbeitskanal 32 ferner eine bis zu dessen Wandfläche reichende Fluidsperre.

Auf der Außenfläche des Arbeitsabschnittes 42 ist eine Sitzscheibe 48 verschiebbar geführt, an welcher das eine Ende einer Schraubenfeder 50 angreift. Deren zweites Ende ist über einen nur schematisch angedeuteten Kraftmesser 52 an der Stirnwand des Arbeitskopfes 40 abgestützt.

Die Sitzscheibe 48 trägt ihrerseits eine Dichtscheibe 54 aus Gummimaterial.

Das Innere des Arbeitsabschnittes 42 steht mit einer Arbeitsleitung 56 des Arbeitskopfes 40 in Verbindung, an welche ein kleiner Druckspeicherraum 58 sowie ein Druckmesser 60 angeschlossen sind.

Die Arbeitsleitung 56 ist über ein federnd in die Schließstellung vorgespanntes 2/2 Magnetventil 62 mit einer Leitung 64 verbunden, die über einen Strommesser 66 und eine durch einen Stellmotor 68 verstellbare Drossel 70 mit dem Ausgang eines 3/3 Magnetventiles 72 verbunden ist. Dessen beide Eingänge sind über einen Druckregler 74 mit dem Ausgang eines Verdichters 76 bzw. über einen Druckregler 78 mit dem Eingang eines Sauggebläses 80 verbunden. Die Regeldrucke der Druckregler 74 und 78 sind über Stellmotoren 82, 84 elektrisch einstellbar.

Die soeben beschriebenen Komponenten 62-84 bilden eine Ver- und Entsorgungseinheit 86, die beim hier zunächst betrachteten Ausführungsbeispiel Luft mit geregeltem Druck bzw. Unterdruck dem Handstück 36 zuführt.

Eine Steuer- und Auswerteeinheit 88 ist mit den elektrischen Ausgangssignalen des Kraftmessers 52, des Druckmessers 60 und des Strommessers 66 verbunden. Weitere Eingangssignale erhält die Steuer- und Auswerteeinheit von einem Tastenfeld 60, um verschiedene Diagnose- und Prüfprogramme auszuführen, die auf einem Massenspeicher 92 abgelegt sind, der z.B. eine Festplatte sein kann.

Zur Steuerung der Intensität der Fluidzufuhr zum Handstück 36 ist auf diesem ein Schieberegler 94 vorgesehen, der mit einem weiteren Eingang der Steuer- und Auswerteeinheit 88 verbunden ist.

Ausgangsseitige Schnittstellen der Steuer- und Auswerteeinheit 88 sind mit den Magnetventilen 62 und 72 sowie mit den Stellmotoren 68, 82 und 84 verbunden.

Zur Anzeige der momentanen Arbeitsbedingungen und von Meßergebnissen ist die Steuer- und Auswerteeinheit 88 mit einem Bildschirm 96 verbunden. Der den behandelnden Arzt momentan am meisten interessierende Arbeitsparameter kann zusätzlich auf einer kleinen LCD-Anzeige 98 ausgegeben werden, die vom Griffteil 38 getragen ist. In der Regel wird dies der durch den Schieberegler 94 eingestellte Fluiddruck sein, für spezielle Anwendungen kann auf die LCD-Anzeige 98 aber auch ein anderer Arbeitsparameter eingegeben werden, z.B. die vom Ausgangssignal des Kraftmessers 52 abgeleitete Eindringtiefe des Arbeitsabschnittes 42 in den Arbeitskanal 32. Diese Strecke ist in Figur 4 mit D angedeutet, wobei die von Sitzscheibe 48 und Dichtscheibe 54 im unbelasteten Zustand eingenommene Stellung gestrichelt als Referenzstellung wiedergegeben ist. Diese Referenzstellung kann durch Anschlagwirkung der Sitzscheibe 48 an einem vom Arbeitsabschnitt 42 getragenen kleinen Anschlag vorgegeben sein.

Das Handstück 36 ist über eine insgesamt mit 99 bezeichnete und nur schematisch angedeutete Drehkupplung mit einem Versorgungskabel 101 verbunden, welches die verschiedenen zur ver- und Entsorgungseinheit 86 und zur Steuer- und Auswerteeinheit 101 führenden Schläuche und Leiter enthält.

Das oben beschriebene Handstück 36 kann zusammen mit seiner Ver- und Entsorgungseinheit 86 und mit der Steuerund Auswerteeinheit 88 das den Arbeitskanal 32 umgebende Gewebe auf Krankheit und Remineralisierungsfähigkeit untersuchen. Hierzu wird es wie folgt eingesetzt:

Über das Tastenfeld 90 wird in die Steuer- und Auswerteeinheit 88 vom Massenspeicher 92 ein Meßprogramm "Porosität-Überdruckmessung" geladen. Durch dieses Programm wird der Druck der am einen Eingang des Magnetventiles 72 anstehende Druckluft auf einen für Gewebe-Porositätsmessungen geeigneten Standardwert eingestellt. Dieser kann typischerweise zwischen 1,2 und 1,5 bar liegen und im Bedarfsfalle durch Betätigen des Schiebereglers 94 zu größeren (bis zu 8 bar) oder kleineren Werten vom Zahnarzt abgeändert werden.

Der Zahnarzt setzt nun das Handstück 36 seitlich am zu untersuchenden Backenzahn 10 an und schiebt den hohlzylindrischen Arbeitsabschnitt 42 progressiv in den Arbeitskanal 32 hinein. Hierbei bleibt die Sitzscheibe 48 und die Dichtscheibe 54 zurück, und letztere wird etwas breitgedrückt, wodurch sich eine Dichtstelle zwischen der Außenfläche des Arbeitsabschnittes 42 und der bukkalen Begrenzungsfläche 14 des Backenzahnes 10 ergibt. Nun kann mit einem Meßvorgang begonnen werden, und dies erkennt die Steuer- und Auswerteeinheit 88 an einem entsprechenden Ausgangssignal des Kraftmessers 52. Sie schaltet daraufhin das Magnetventil 72 in diejenige Stellung, in welcher der Ausgang des Druckreglers 74 mit der auslaßseitigen Arbeitsöffnung des Magnetventiles 72 verbunden ist. Damit steht am Einlaß des Magnetventiles 62 Druckluft unter dem gewünschten Druck an.

Die Steuer- und Auswerteeinheit 88 öffnet nun in regelmäßigen Abständen das Magnetventil 62, so daß der Innenraum des Arbeitsabschnittes 42, der Arbeitskanal 32 und das ihn umgebende Gewebe sowie der Druckspeicher 58 mit Druckluft gefüllt werden. Der Auf-Zustand des Magnetventiles 62 wird für eine Zeitspanne aufrechterhalten, die zur vollständigen Füllung des Druckspeichers 58, des Arbeitsabschnittes 42 und des mit diesem kommunizierenden Abschnittes des Arbeitskanales 32 ausreicht. Hierfür kann entweder eine erfahrungsgemäß ausreichende zeitspanne gewählt werden oder durch die Steuer- und Auswerteeinheit 88 festgestellt werden, wann das Ausgangssignal des Druckmessers 62 konstant bleibt. Nun wird das Magnetventil 62 geschlossen, und die Steuerund Auswerteeinheit 88 zeichnet den Abfall des Ausgangssignales des Druckmessers 60 auf. Bei guter Abdichtung zwischen dem außenliegenden Ende des Arbeitskanales 32 und des Arbeitsabschnittes 42 ist der Druckabfall, den der Druckmesser 60 feststellt, auf Luft-Leckströme zurückzuführen, welche über den kranken Gewebebereich 24 zur Umgebungsatmosphäre hin erfolgen. Je fortgeschrittener die Zerstörung dieses Gewebebereiches ist, umso schneller erfolgt der Druckabbau.

Aus früheren Messungen und Eichmessungen ist bekannt, ab welcher Druckabnahme das kranke Gewebe nicht mehr zu einer Remineralisierung fähig ist. Ist kein Druckabfall festzustellen oder der Druckabfall langsam, kann eine Remineralisierungs-Therapie mit guten Brfolgsaussichten erfolgen.

Die oben skizzierte Messung läßt sich gleichermaßen unter Unterdruckbeaufschlagung der Arbeitsleitung 56 durchführen, analog wie oben für Überdruckbeaufschlagung beschrieben. In diesem Falle stellt die Steuerund Auswerteeinheit 88 in einem vom Massenspeicher 92 eingelesenen Programm "Porosität-Unterdruckmessung" einfach das Magnetventil 72 in die andere Arbeitsstellung, in welcher der Druckregler 78 mit dem Auslaß des Magnetventiles 72 verbunden ist. Die Steuer- und Auswerteeinheit 88 registriert dann den nach einem Schließen des Magnetventiles 62 am Druckmesser 66 eintretenden Druckanstieg. Für Standard-Untersuchungen haben sich Unterdruckwerte (Druckabsenkungen gegenüber Normaldruck) von zwischen 100 und 500 mbar bewährt. Es versteht sich, daß man diese Druckwerte im Einzelfalle noch weiter erhöhen oder erniedrigen kann, wie dies im Hinblick auf die Porosität des kranken Gewebebereiches und im Hinblick auf die Meßgenauigkeit im Einzelfalle am günstigsten ist.

In Abwandlung des oben beschriebenen Ausführungsbeispieles kann man als zur Messung der Porosität des kranken Gewebebereiches 24 verwendetes Gas anstelle von Luft auch ein inertes Gas oder ein anderes Gas verwenden, welches zugleich eine therapeutische Wirkung entfaltet, z.B. chlorhaltige Luft.

In weiterer Abwandlung des unter Bezugnahme auf Figur 4 beschriebenen Ausführungsbeispieles kann man anstelle eines Gases zur Messung der Porosität des kranken Gewebebereiches 24 auch eine Flüssigkeit verwenden, z.B. Wasser oder eine physiologische Kochsalzlösung, oder eine therapeutisch wirksame Lösung.

In diesem Falle stellt dann das Bauteil 76 eine Flüssigkeits-Förderpumpe, das Bauteil 80 eine Flüssigkeits-Saugpumpe dar. Die Förderpumpe 76 saugt die Diagnoseflüssigkeit aus einem Vorratsbehälter 100 an, der in Figur 4 gestrichelt eingetragen ist. Die Steuer- und Auswerteeinheit 88 bestimmt nun die Porosität des kranken Gewebebereiches 24 ausgehend vom Ausgangssignal des Strommessers 66. Das Ausgangssignal des Druckmessers 60 kann zusätzlich verwendet werden, um eine Anzeige dafür zu haben, wann Restluft im Handstück und im Gewebe verdrängt ist. Bei einer vereinfachten Version eines nur mit Diagnoseflüssigkeiten arbeitenden Handstückes kann man den Druckspeicherraum 58 und den Druckmesser 60 auch weglassen. Das in Figur 4 gezeigte Handstück ist aber gleichermaßen zur Messung der Gewebeporosität mittels gasförmiger und flüssiger Fluids geeignet.

Arbeitet die Steuer- und Auswerteeinheit 88 in Abhängigkeit vom Ausgangssignal des Strommessers 66, so ist der im stationären Zustand vom Strommesser 66 gemessene Flüssigkeitsstrom, der laufend über den kranken Gewebebereich 24 in die Umgebung des Zahnes austritt, ein Maß für die Stärke der Erkrankung bzw. den Grad der Demineralisation bzw. der vorhandenen Porositäten bzw. Kavitäten.

In einer kombinierten Diagnose-Betriebsart kann man auch die das zeitliche Verhalten des vom Strommesser 66 gemessenen Flüssigkeitsstromes betrachten, wobei die bis zur Erzielung eines stationären Flüssigkeitsstromes erhaltenen größeren Stromstärken einer anfänglichen Verdrängung von Luft aus dem durch das Diagnosegerät abgedichteten Arbeitskanal entspricht. Diese Verdrängung erfolgt ebenfalls über den kranken Gewebebereich bzw. in diesen hinein.

Dadurch, daß man Flüssigkeiten unterschiedlicher Viskosität verwendet, kann man den Meßbereich des in Figur 4 gezeigten Diagnosegerätes verlagern: niederviskose Flüssigkeiten wie Wasser eignen sich gut zur Messung von weniger porösen kranken Gewebebereichen, während man für stärker poröse Gewebebereiche Diagnoseflüssigkeiten mit höherer Viskosität verwenden kann.

Den Diagnoseflüssigkeiten können zusätzlich Markiersubstanzen zugesetzt sein, die von gesundem und krankem Gewebe unterschiedlich aufgenommen werden und eine optische und/oder analytische Unterscheidung von krankem und gesundem Gewebe erleichtern. Hierzu gehören z.B. Farbstoffe wie Fuchsin, wobei Letzteres z.B. als 0,5%ige basische Lösung in Propylenglykol verwendet werden kann.

Weitere geeignete Markiersubstanzen sind Elektrolyte, Röntgenkontrastmittel, Radionukleide, etc.

Wird zur Erzeugung des Arbeitskanales ein Hohlbohrer verwendet (konventionelles Bohrgerät mit rotierenden diamantierten Hohlbohrern, wie ozillierende Präparationsgeräte), so können die hohlen Bohrer zugleich als Fluid zu- oder abführende Sondenabschnitte dienen, wenn über eine geeignete Ausbildung der Bohrwerkzeugaufnahme eine Verbindung des Innenraumes des Hohlbohrwerkzeuges mit einer Fluidquelle bzw. einer Fluidsenke hergestellt wird. In diesem Falle kann dann das Bohrgerät zugleich auch ein Diagnose- und Behandlungsgerät darstellen, wie es obenstehend unter Bezugnahme auf die Figur 4 beschrieben wurde.

Auf diese Weise kann man besonders bequem den Arbeitskanal durch schrittweises Vorschieben des Bohrwerkzeuges in Richtung der vermuteten Läsion vortreiben und gleichzeitig ohne Wechsel des Gerätes nach jedem Inkrement des Vorschubs die fluiddynamische Diagnostik zur Bestimmung der Gesundheit des Gewebes durchführen. Hierdurch wird das Arbeiten insgesamt stark erleichtert.

Ggf. muß zur Untersuchung auf Druckmaxima der "natürliche Defektzugang" oberflächlich gereinigt oder z.B. durch Einbringen eines Holzkeiles von der Approximaloberfläche des Nachbarzahnes, separiert werden. In diesem Fall ermöglicht die verwendung einer Ätzlösung zur Untersuchung der Druckgradienten eine Konditionierung der defektbildenden demineralisierten Zahnhartgewebe bzw. der begrenzenden intakten Zahnhartgewebe und schafft so einen verbesserten Diffusionsquerschnitt zur Eindiffusion von Speichel und z.B. therapeutischen Wirkstoffen, wie z.B. den üblichen in der Zahnheilkunde verwendeten Fluoridpräparaten. Es können jedoch auch die Qualität der Diffusionsbarriere, z.B. durch Verlegung von Porositäten, beeinflussende Adhäsive verwendet werden. Gleichzeitig werden die im Defekt lokalisierten Bakterien und ggf. smear layer-Schichten wirksam abgetötet bzw. entfernt. Es bietet sich an, die Druckwerte und ggf. Flüssigkeitsfüllmengen und/oder die endoskopischen Bilder und/oder weitere Befunde zu dokumentieren, um im Verlauf der Kontrolle des. Erfolgs der Remineralisationsbehandlung oder ggf. induzierten Remineralisation während des Monitorings Vergleichsgrößen zu haben und somit Trends der Reoder weiterer Demineralisation bevorzugt ohne weiteres Röntgenbild reproduzierbar zu erkennen.

Das in Figur 4 gezeigte Gerät kann auch als Behandlungsgerät eingesetzt werden, um in porösem krankem Gewebe einen Flüssigkeitsaustausch durchzuführen, insbesondere fluoridhaltige Flüssigkeiten in diese Gewebebereiche hineinzudrücken. In Ergänzung können auch andere Behandlungsflüssigkeiten, welche das kranke Gewebe für eine Fluoridisierung vorbereiten, mit dem Handstück nach Figur 4 in das kranke Gewebe gedrückt werden und nach vorgegebener Behandlungszeit wieder aus diesem Gewebe herausgesaugt oder durch das Gewebe hindurchgedrückt werden. Hierzu werden einfach Vorratsbehälter 100 verwendet, die anstelle einer Diagnoseflüssigkeit eine Behandlungsflüssigkeit enthalten.

Derartige Behandlungsflüssigkeiten können z.B. sein:
a) Spülflüssigkeiten: diese holen bei der Erzeugung des Arbeitskanales oder auf Grund der Erkrankung erhaltene Gewebebruchstücke und Bakterien aus dem kranken Gewebebereich heraus. Erfolgt das Zuführen der Spülflüssigkeiten unter Oszillation, können hierdurch auch die Zellwände von Bakterien im kranken Gewebebereich zerstört werden oder die Bakterienoberflächen derart geschädigt werden, daß diese keine destruierenden Stoffwechselleistungen mehr vollbringen können. Bei raschem Druckwechsel können Hartsubstanz abtragende Kavitationseffekte erhalten werden.
b) Desinfektionslösungen, wie z.B. Chlorhexidinlösungen: diese zerstören chemisch im kranken Gewebebereich angefundene Bakterien.
c) Adhäsive: (z.B. Carboxylgruppen enthaltende Lösungen, modifizierte Polyacrylsäurelösungen, Lösungsgemische beinhaltend Acrylatmono- oder Polymere oder Lösungsmittel-(z.B. Aceton-)haltige Monomer- oder Polymerlösungen etc.)
d) Organische Gewebereste auflösende Lösungen, z.B. Natriumhypochloridlösungen in Konzentrationen unter 6 Gew.-%, bevorzugt unter 3 Gew. -%, nochmals bevorzugt um etwa 1 Gew.-% und N-Monochlor-DL-2-Aminobutyrat enthaltende Lösungen.
e) Ätzlösungen: hierzu gehören wässrige Kalziumchelatbildner, z.B. EDTA-Lösungen unter 50 Gew.-%, bevorzugt um 20 Gew.-%. Diese Lösungen ermöglichen ein chemisches Abtragen weitestgehend demineralisierter Gewebe und anorganische und organische Säuren.
f) Inerte Partikel enthaltende Flüssigkeiten: diese tragen kleine inerte Partikel in die poröse Struktur des kranken Gewebes, wodurch diese abgedichtet wird. Derartige Partikel können auch in den Grenzflächen verkeilt werden und können in das Reinfiltrationsvolumen mit eingebaut werden, um so zusätzliche Hafteffekte des reinfiltrierten Materials an der Oberfläche der im Hartgewebe verkeilten Partikel zu begründen. Derartige Partikel können auch zur Reinigung, beschleunigten Abtragung, Präparation oder Konditionierung des kranken Gewebebereiches nützlich sein, wenn sie unter Druck-Wechselbelastungen oder unter Ultraschalleinwirkung gegen das kranke Gewebe bewegt werden.
   Geeignete inerte Partikel sind Glaspartikel, Kalziumphosphatpartikel, Hydroxylapatit- oder Fluorapatitpartikel, Kalziumfluoridpartikel, Salzpartikel, Carbonat partikel, Flußspatpartikel, Keramikpartikel, Kunststoffpartikel oder Kompositpartikel. Dabei werden Teilchendurchmesser zwischen 0,5 und 2 µm oder auch bis etwa 5µm bevorzugt, um die oben angesprochene Abdicht- und Brückenfunktion zu erfüllen, wobei für stärker geschädigte kranke Gewebebereiche und zur mechanischen Bearbeitung von Gewebe auch Teilchenpartikel mit größerer mittlerer Korngröße geeignet sind (5-10 µm bis hin zu 20, 50 oder 100 µm).
   Die Wahl der Partikelgrößen erfolgt je nach Anwendung:
   Ist man an den abrasiven Eigenschaften der Teilchen interessiert, wählt man Teilchen mit mittleren oder größeren Durchmessern aus der oben angegebenen Skala. Möchte man dagegen offene Dentinkanälchen im Sinne der Herstellung einer internen Diffusionsbarriere, verlegen (sei dies außen an sensiblen Zahnhälsen oder nach innen bei der Pulpa zugewandten Defektoberflächen) verwendet man Partikelgrößen, die den kleineren oben genannten Größenbereichen entsprechen.
   In beiden Fällen können derartige inerte Partikel Haftsubstrate bilden.
   Gegebenfalls können auch Gemenge verschiedener Partikel verwendet werden, die unterschiedlichen mittleren Teilchendurchmesser aufweisen, wobei diese unterschiedlichen mittleren Teilchendurchmesser im Hinblick auf optimale Funktion für verschiedene der oben angesprochenen Teilaufgaben gewählt sind.
f) Pulpawirksame Lösungen: diese Lösungen enthalten Wirkstoffe, die Heilungsprozesse in der Pulpa unterstützen können und werden verwendet, wenn der zu remineralisierende kranke Gewebebereich der Pulpa benachbart ist. Beispiele für solche Wirkstoffe sind BMP (Bone Morphogenetic Proteins) sowie Kalziumhydroxid (Ca(OH)₂) zur Anregung der Hartgewebebildung und K-und Sr-Verbindungen zur Desensibilisierung der Pulpa.
g) Remineralisationsfördernde Flüssigkeiten: diese Flüssigkeiten enthalten Wirkstoffe, welche die Remineralisation unterstützen. So verschieben z.B. Fluoride das De-/Remineralisationsgleichgewicht zugunsten der Remineralisation, auch in saurer Umgebung (bis pH 4,5). Auch gezielte bakteriostatische oder bakteriozide Wirkungen, wie sie z.B. Chlorhexidin bereitstellt, unterstützen die Remineralisation (Elimination der säurebildenden Bakterien). Die Remineralisation selbst erfolgt vorzugsweise durch im Speichel gelöste Mineralien, die über den "natürlichen Karieszugang" zur Läsion gelangen.
h) Bei der Reinfiltration verwendete Flüssigkeiten, Gele oder Pasten: Diese bilden nach dem Aushärten in den porösen Strukturen und/oder Kavitationen der Läsion und/oder in durch Präparation erzeugten Hohlräumen und/oder in benachbarten Gewebestrukturen ein Hartgewebe-Ersatzmaterial.

Die oben genannten Behandlungsflüssigkeiten können in der gewünschten Reihenfolge nacheinander jeweils in einem Vorratsbehälter 100 von der Förderpumpe 76 angesaugt und in den Arbeitskanal 32 und damit in den kranken Gewebebereich 24 gedrückt werden.

Wo ein kranker Gewebebereich in Strömungsverbindung mit der Zahnaußenfläche steht, reicht es, die Behandlungsflüssigkeit ständig unter Druck zu halten; die Behandlungsflüssigkeit strömt dann durch den kranken Gewebebereich zur Zahnaußenfläche.

Wo eine derartige Strömungsverbindung nicht besteht, weil der kranke Gewebebereich von gesundem Gewebe umgeben ist, unterstützt man den Flüssigkeitsaustausch zwischen dem kranken Gewebe und dem Arbeitsabschnitt 42 des Handstückes 36 dadurch, daß man ein Arbeitsprogramm "Behandlung-Drücken und Saugen" der Steuer- und Auswerteeinheit 88 auswählt, bei welchem das Magnetventil 72 intermittierend zwischen seinen beiden Arbeitsstellungen umgeschaltet wird, wodurch Behandlungsflüssigkeit intermittierend in das kranke Gewebe gedrückt und aus diesem wieder herausgesogen wird. Zurückgesaugte Flüssigkeitsmengen werden von der Saugpumpe 80 in eine Sammelbehälter 102 abgegeben.

Die verschiedenen oben angesprochenen Flüssigkeiten werden somit für folgende Zwecke verwendet:

Diagnostik: Es wird geprüft, ob eine Läsion zur Zahnoberfläche hin permeabel ist ("natürlicher Karieszugang"), und wenn ja, ob die Permeabilität so groß ist, daß eine Remineralisierung nicht mehr möglich ist.

Behandlung zur Unterstützung der Remineralisation: Eine Meßstelle umgebendes remineralisierbares Gewebe bildet eine gute Diffusionsbarriere. Durch Einbringen einer Behandlungsflüssigkeit unter kontinuierlichem Druck erfolgt über längere Zeit hinweg eine Diffusion der in der Behandlungsflüssigkeit enthaltenen Wirkstoffe in das umgebende Gewebe, wodurch dieses für eine bessere Remineralisation vorbereitet wird. Der Austausch zwischen Gewebeflüssigkeit und Behandlungsflüssigkeit kann durch Pumpwirkung unterstützt werden, indem die Behandlungsflüssigkeit alternierend unter Überdruck und Unterdruck gesetzt oder unter Schall- oder Ultraschalleinwirkung appliziert wird.

Behandlung zur Vorbereitung einer Reinfiltration: Durch rasche Druckwechselbelastung oder "Wasserstrahlwirkung", durch Ultraschalleinwirkung (ggf. unterstützt durch abrasive Partikel) werden irreversibel zerstörte Gewebeanteile innerhalb des Läsionsvolumens ausgeräumt. Dabei dient das Behandlungsmedium als Koppel- und Transportmedium. Soweit das Behandlungsmedium zusätzlich Wirkstoffe enthält, erfolgt auch ein Einbringen der Wirkstoffe in die verbleibende Gewebestruktur, wo diese dann ihre chemische oder biologische Wirkung entfalten können.

Spülwirkung: Bei großer Durchlässigkeit der Diffusionsbarriere kann man die Behandlungsmedien auch kontinuierlich durch die Läsion hindurchdrücken (reine Spül- bzw. Abführ- bzw. Trockungswirkung).

Reinfiltration: Es wird ein flüssiges aushärtbares Material eingebracht, welches ggf. noch zurückgebliebene Reste anderer Behandlungsmedien verdrängt und nach dem Aushärten ein Zahnersatzmaterial bildet.

Zur Vereinfachung eines mehrstufigen Behandlungsverfahrens kann man auch mehrere der Behandlungsflüssigkeiten gemischt in einen Vorratsbehälter 100 geben und gemeinsam dem kranken Gewebebereich zuführen.

Es versteht sich, daß ein Vorratsbehälter 100 für Behandlungsmedium in der Praxis kein offenes Gefäß ist, vielmehr ein steriler Beutel, der an die als Kanüle ausgebildete Ansaugleitung der Förderpumpe 76 angeschlossen wird.

Aus der obenstehenden Beschreibung im Zusammenhang mit Figur 4 ist ersichtlich, daß das dort gezeigte Gerät ein kombiniertes Diagnose- und Behandlungsgerät ist, welches sich zur Diagnose wahlweise mit gasförmigen und flüssigen Diagnosemedien und zur Behandlung wahlweise mit gasförmigen und flüssigen (oder wenigstens kurzzeitig in fließfähigem Zustand versetzten) Behandlungsmedien eignet.

Die Umsetzung der Druckanstiegen, Druckabfällen oder Flüssigkeitsströmen und Änderungen derselben zugeordneten elektrischen Ausgangssignale in ein der Stärke der Erkrankung des kranken Gewebebereiches 24 entsprechende elektrische Signale, die auf dem Bildschirm 96 ausgegeben werden, erfolgt durch die Steuer- und Auswerteeinheit 88. Hierbei kann die Steuer- und Auswerteeinheit zum einen gemäß Algorithmen arbeiten, welche aus den Strömungsgesetzen abgeleitet sind; die Steuer- und Auswerteeinheit kann aber stattdessen oder zusätzlich auch nach Zuordnungstafeln arbeiten, die anhand von früheren Untersuchungen oder Messungen an Testmaterialien gewonnen wurden. Dabei können unterschiedliche Zuordnungstabellen verwendet werden, die unterschiedlichen Zahnarten (Backenzähne, Schneidezähne), dem Geschlecht des Patienten und dem Alter des Patienten Rechnung tragen. Diese Tabellen sind auf dem Massenspeicher 92 abgelegt, und die jeweils benötigte wird für eine spezielle Messung von der Steuerund Auswerteeinheit 88 geladen.

Ferner enthält der Massenspeicher 92 unterschiedliche Meßprogramme (z.B. für gasförmige Diagnosemedien und flüssige Diagnosemedien unterschiedlicher Viskosität). Schließlich sind auf dem Massenspeicher 92 auch die Behandlungsprogramme abgelegt, welche die Steuerung der Magnetventile 62, 72 und die Steuerung der Arbeitsdrucke über die Stellmotoren 82, 84 sowie eine Strombegrenzung durch die Drossel 70 durch den Stellmotor 68 für die verschiedenen Behandlungsmedien vorgeben.

Ist der kranke Gewebebereich 24 sehr nahe bei der Zahnoberfläche angeordnet und ist die Schädigung des Gewebes schon so stark, daß ein erheblicher Leckpfad zur Zahnoberfläche gebildet wird, treten durch das kranke Gewebe bei Flüssigkeitsbeaufschlagung des Arbeitskanales 32 größere Flüssigkeitsmengen zur Zahnoberfläche hin aus. Um zu verhindern, daß diese Flüssigkeitsmengen auch auf den benachbarten Zahn einwirken und in den Mund des Patienten gelangen, kann man gemäß Figur 11 zwischen die beiden Zähne ein Saugteil 200 einschieben, wobei gegebenenfalls durch einen Keil 202 der Zahnabstand vorübergehend vergrößert wird. Das Saugteil 200 ist ein aus Gummi oder Kunststoff gefertigtes Hohlteil, welches über einen Anschlußstutzen 204 mit einer Saugleitung 206 verbunden ist. In der in der Zeichnung links gelegenen Hauptfläche ist das Saugteil 200 mit einer Saugöffnung 208 versehen, welche neben den kranken Gewebebereich 24 gestellt wird.

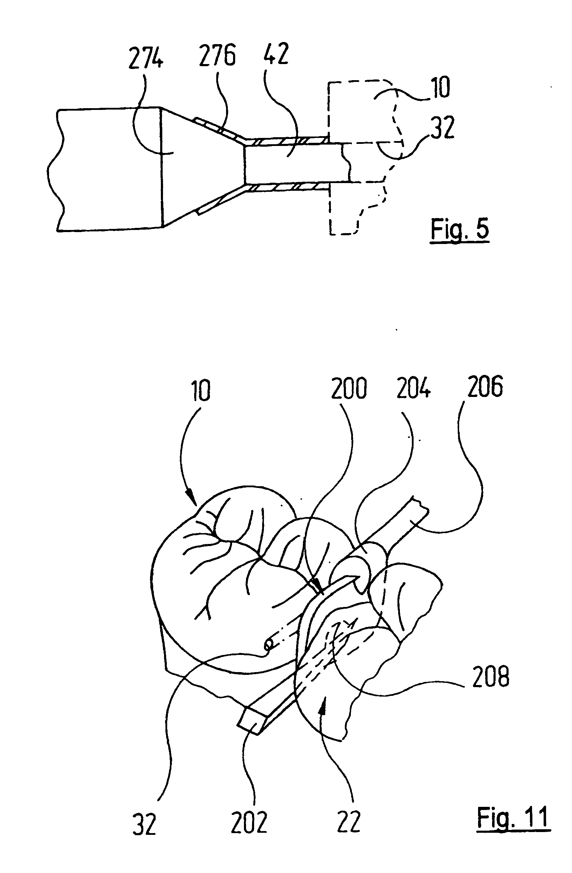

Figur 5 zeigt eine abgewandelte Dichtstelle zwischen einem Arbeitsabschnitt 42 eines Diagnose- oder Behandlungsgerätes und einem in einem Zahn erzeugten Arbeitskanal 32. Der Arbeitsabschnitt 42 ist an seinem vom freien,Ende abgelegenen Ende durch eine kegelförmige Dichtschulter 274 begrenzt. Auf den Arbeitsabschnitt 42 ist ein zylindrisches Dichtelement 276 aufgeschoben, welches durch ein Stück Sililkonschlauch gebildet sein kann. Beim axialen Hineinbewegen des Arbeitsabschnittes 42 in den Arbeitskanal 32 kommt die in Figur 5 rechts gelegene Stirnfläche des Dichtelementes 276 in Anlage an die Oberfläche des Zahnes 10, der in Figur 5 gestrichelt angedeutet ist. Beim weiteren Einschieben des Arbeitsabschnittes 42 wird das Dichtelement 276 auf dem Arbeitsabschnitt 42 in Figur 5 nach links bewegt und sein von der Zahnoberfläche abgelegener Endabschnitt gleitet unter radialer Aufweitung auf die kegelförmige Dichtschulter 274, wie aus Figur 5 ersichtlich.

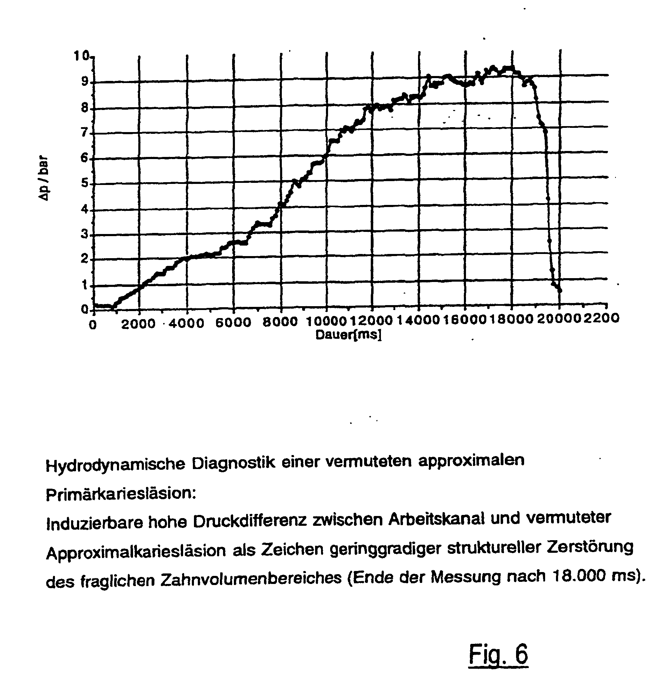

In den Figuren 6 bis 9 sind Druckverlaufskurven wiedergegeben, wie sie an unterschiedlich stark geschädigten Zähnen in zum kranken Gewebebereich führenden Arbeitskanälen gemessen wurden. Man erkennt aus den verschiedenen Meßdiagrammen, daß sich wenig geschädigte Zahnhartgewebe, mäßig geschädigte und möglicherweise noch durch Remineralisierung heilbare Zahnhartgewebe und stark geschädigte und nur noch durch Füllung behandelbare Zahnhartgewebe deutlich im Druckverlauf unterscheiden.

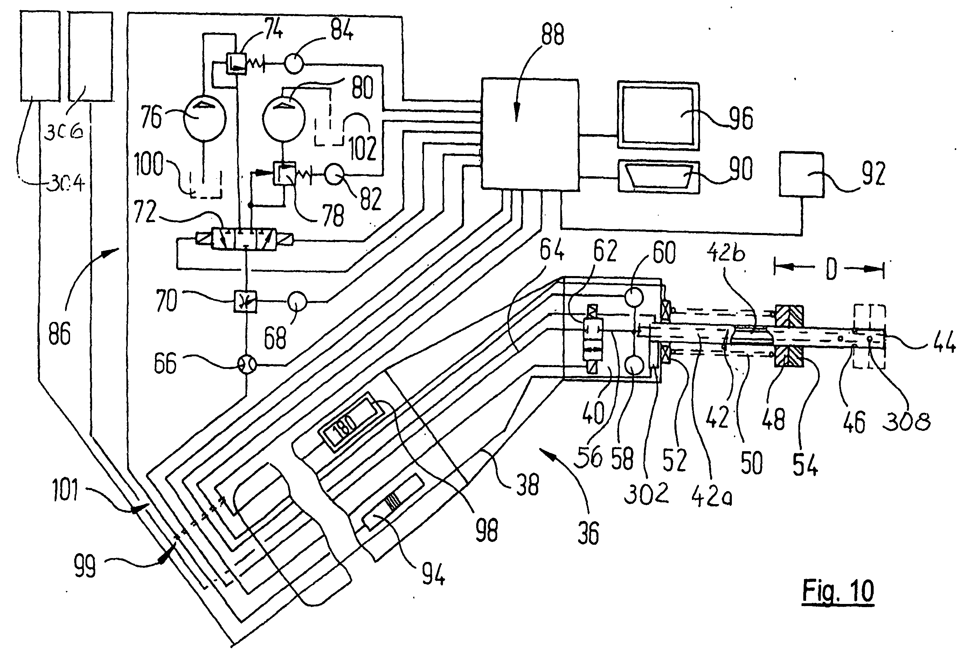

Das in Figur 10 dargestellte Arbeitsgerät kann gleichzeitig zum Bohren eines Arbeitskanales, zu Diagnoseund ggf. zur Behandlung verwendet werden.

Der Arbeitsabschnitt 42 umfaßt ein außenliegendes rohrförmiges Bohrwerkzeug 42a, welches mechanisch an einen Ultraschallschwinger 302 angekoppelt ist, der durch eine versorgungseinheit 304 gespeist wird. Innerhalb des rohrförmigen Bohrwerkzeuges 42a ist ein Leitrohr 42b angeordnet. Dieses begrenzt zusammen mit dem Bohrwerkzeug 42a einen Ringraum, der mit einer steuerbaren Schleifmittelquelle 306 verbunden ist, die einen Vorratsbehälter und eine Pumpe für Schleifmittel enthaltende Arbeitsflüssigkeit umfassen kann.

Die Schleifmittelflüssigkeit kann durch die Ringfläche zwischen Bohrwerkzeug 42a und Leitrohr 42b und zusätzlich über in der Mantelfläche des Bohrwerkzeuges 42a vorgesehene Öffnungen 308 austreten.

Das Innere des Leitrohres 42b ist über das Magnetventil 62 und das Magnetventil 72 mit der Saugpumpe 80 verbindbar, um verbrauchte Schleifmittel enthaltende Arbeitsflüssigkeit abzuziehen.

Man erkennt, daß man mit dem in Figur 30 gezeigten Gerät durch Ultraschalleinwirkung (unterstützt durch Schleifmittel) wohldefinierte Arbeitskanäle in einem Zahn erzeugen kann, gleichzeitig aber das Gerät auch laufend zu Diagnosezwecken und nach Abschluß der Diagnose auch zur Behandlung verwenden kann.

In Abwandlung kann man das Leitrohr 42b ebenfalls an den Ultraschallschwinger 302 oder einen gesonderten Ultraschallschwinger (nicht gezeigt) ankoppeln, um das Fördern von viskosen und/oder Partikel enthaltenden Medien durch das dünne Leitrohr selbst und/oder durch den Ringraum zwischen Bohrwerkzeug 42a und Leitrohr 42b zu begünstigen.

Obenstehend wurden bei der Beschreibung der Diagnosemedien nur diejenigen Bestandteile detailliert erläutert, welche für die Funktion in besonderer Weise wichtig sind. Es versteht sich, daß man darüber hinaus die Diagnosemedien zusätzlich bezüglich ihrer hydrophoben/hydrophilen Eigenschaften durch entsprechende Zusätze einstellen kann und insbesondere auch bezüglich ihrer Viskosität einstellen kann. Entsprechend inerte Verdickungsmittel sind dem Fachmann bekannt.

## Patentansprüche

1. Gerät zur Bestimmung der Remineralisierungsfähigkeit von Hartgewebe, insbesondere kariösem Zahngewebe, mit einer Meßeinrichtung (46, 60, 86, 88) zum lokalen Messen einer physikalischen Eigenschaft des Hartgewebes, die mit einem Meßkopf (40) zusammenarbeitet, der einen in einen Arbeitskanal (32) im Hartgewebe einführbaren stabförmigen Sondenabschnitt (42), und ein Dichtelement (54) aufweist, welches mit einem Abschnitt der Gewebeoberfläche zusammenarbeitet, **dadurch gekennzeichnet, daß** ein die Porosität des Hartgewebes ermittelndes Meßelement (60; 66) mit dem Sondenabschnitt (42) in Verbindung steht, und daß der Meßkopf (40) mit einer unter von Normaldruck verschiedenem Druck stehenden Diagnosefluidquelle (76; 80) in Verbindung steht und die Meßeinrichtung Zur Messung der Diagnosefluid-Leckage durch das zu untersuchende Gewebe ausgebildet ist.

2. Gerät nach Anspruch 1, **gekennzeichnet durch** einen Stellungsgeber (48-52) für die Axialstellung des Sondenabschnittes (42) bezüglich einer Begrenzungsfläche des Gewebes.

3. Gerät nach Anspruch 2, **dadurch gekennzeichnet,**
**daß** der Sondenstellungsgeber (48-52) ein mit der Gewebeoberfläche zusammenarbeitendes Eingangsteil (48) aufweist, welches axial verschiebbar auf dem Sondenabschnitt (42) angeordnet ist und über eine Feder (50) an einen feststehenden Geräteabschnitt abgestützt ist, welcher ein Kraft- oder Verformungssensor (52) zugeordnet ist.

4. Gerät nach Anspruch 3, **gekennzeichnet durch** eine Auswerteeinheit (88), welche mit dem Ausgangssignal des Sondenstellungsgebers (48-52) und dem Ausgangssignal des Meßelementes beaufschlagt ist und die Ausgangssignale des letzteren, die für unterschiedliche Stellung des Sondenabschnittes (42) erhalten werden, miteinander vergleicht, wozu die jeweils für die verschiedenen Sondenstellungen erhaltenen Meßsignale in Abhängigkeit vom Ausgangssignal des Sondenstellungsgebers (48-52) abgespeichert werden.

5. Gerät nach einem der Ansprüche 1-4, wobei das Meßelement eine bezogen auf die Achse des Sondenabschnittes (42) radiale Meßachse aufweist, **dadurch gekennzeichnet, daß** der Sondenabschnitt (42) drehbar (118-122) auf dem Meßkopf (40) angeordnet ist.

6. Gerät nach einem der Ansprüche 1-5, **dadurch gekennzeichnet, daß** der Meßkopf (40) an einem Griffteil (36) angebracht ist, wobei er vorzugsweise um dessen Längsachse drehbar ist.

7. Gerät nach Anspruch 6, **dadurch gekennzeichnet, daß** die Längsachse des Griffteiles (36) und die Achse des Meßkopfes (40) gegeneinander geneigt sind und vorzugsweise einen Winkel zwischen etwa 30° und etwa 90°, nochmals vorzugsweise von etwa 60° einschließen.

8. Gerät nach Anspruch 6 oder 7, **dadurch gekennzeichnet, daß** das Griffteil (36) über eine Drehkupplung (99) mit einem Versorgungskabel (101) verbunden ist.

9. Gerät nach einem der Ansprüche 1-8, **dadurch gekennzeichnet, daß** Mittel (94) zur Einstellung der Stärke der Zufuhr von Diagnosefluid vorgesehen sind.

10. Gerät nach Anspruch 9, **dadurch gekennzeichnet, daß** die Einstellmittel eine vom Griffteil (36) getragene Eingabeeinrichtung (94) umfassen.

11. Gerät nach Anspruch 9 oder 10, **dadurch gekennzeichnet, daß** die Einstellmittel eine Programmsteuerung (88) umfassen.

12. Gerät nach Anspruch 11, **dadurch gekennzeichnet, daß** die Programmsteuerung so arbeitet, daß die Zufuhr von Diagnosefluid so erregt gesteuert wird, daß am Meßelement (60) ein vorgegebenes Ausgangssignal erhalten wird.

13. Gerät nach einem der Ansprüche 1-12, **dadurch gekennzeichnet, daß** der Sondenabschnitt (42) zylindrisch ist.

14. Gerät nach einem der Ansprüche 1-13, **dadurch gekennzeichnet, daß** das Fluid ein Gas ist und daß der Meßkopf (40) über ein Schaltventil (62), welches eine Schließstellung aufweist, mit der Fluidquelle (76; 80) verbindbar ist und an den Meßraum, welcher durch den Meßkopf (40) und das Gewebe begrenzt wird, ein Druckmesser (60) angeschlossen ist.

15. Gerät nach Anspruch 14, **dadurch gekennzeichnet, daß** der Meßkopf (40) einen mit dem Meßraum verbundenen Druckspeicher (58) aufweist.

16. Gerät nach einem der Ansprüche 1-13, **dadurch gekennzeichnet, daß** das Diagnosefluid eine Flüssigkeit ist und daß in der von der Fluidquelle (76) zum Meßkopf (40) führenden Versorgungsleitung ein Strommesser (66) angeordnet ist.

17. Gerät nach Anspruch 16, **dadurch gekennzeichnet, daß** der Durchflußmesser (66) ein Kapillarrohr und Mittel zum Einspeisen von Gasblasen in das stromabseitige Ende des Kapillarrohres aufweist.

18. Gerät nach einem der Ansprüche 1-17 in Verbindung mit Anspruch 3, **dadurch gekennzeichnet, daß** das Dichtelement (54) von der freien Stirnfläche des Eingangsteiles (48) des Sondenstellungsgebers (48-52) getragen ist.

19. Gerät nach einem der Ansprüche 1-17, **dadurch gekennzeichnet, daß** der Sondenabschnitt (42) ein am Ende geschlossenes (44) zylindrisches Rohr ist, welches in seiner Umfangswand mindestens eine Fluid-Durchtrittsöffnung aufweist.

20. Gerät nach Anspruch 19, **dadurch gekennzeichnet, daß** der Sondenabschnitt (42) an seinem vom freien Ende abgelegenen Ende eine sich in radialer Richtung erweiternde, vorzugsweise kegelförmige Schulter (274) aufweist und daß ein Dichtschlauch (276) auf der Außenseite des Sondenabschnittes (42) angeordnet ist und mit seinem vom freien Ende des Sondenabschnittes (42) abgelegenen Ende auf die Dichtschulter (274) unter Aufweitung aufschiebbar ist.

21. Gerät nach einem der Ansprüche 1-20, **dadurch gekennzeichnet, daß** der Meßkopf (40) über ein Umschaltventil (72) alternierend mit einer Überdruck-Fluidquelle (76) und einer Unterdruck-Fluidquelle (80) verbindbar ist.

22. Gerät nach Anspruch 21 in Verbindung mit Anspruch 16, **gekennzeichnet durch** Mittel (102) zum Auffangen des aus dem Meßkopf (40) abgesaugten Flüssigkeitsvolumens.

23. Gerät nach einem der Ansprüche 1-22, **dadurch gekennzeichnet, daß** die Diagnosefluidquelle (76; 80) eine Überdruckquelle ist, welche Diagnosefluid mit einem Überdruck von 0,1 bis 0,8 bar, bevorzugt 0,1 bis 0,5 bar bereitstellt.

24. Gerät nach einem der Ansprüche 1 bis 23, **gekennzeichnet durch** Mittel zum Beaufschlagen des Diagnosefluids mit Schall oder Ultraschall.

25. Gerät nach Anspruch 24, **gekennzeichnet durch** ein mit Schall oder Ultraschall beaufschlagtes Zuführteil (42b) für das Diagnosesfluid, welches in einen Arbeitkanal (32) einführbar ist.

26. Gerät nach Anspruch 25, **dadurch gekennzeichnet, daß** das Zuführteil (42b) hohl ist und sein Innenraum mit einer Unterdruckquelle (80) verbindbar ist.

27. Gerät nach einem der Ansprüche 1-26, **dadurch gekennzeichnet, daß** das Diagnosefluid eine auf kariöses Gewebe selektiv ansprechende Markiersubstanz enthält.

28. Gerät nach Anspruch 27, **dadurch gekennzeichnet, daß** die Markiersubstanz ein Färbemittel wie Fuchsin, ein Röntgenkontrastmittel, ein Radionukleid oder einen Elektrolyten umfaßt.

29. Gerät nach einem der Ansprüche 1-28, **dadurch gekennzeichnet, daß** das Diagnosefluid inerte Partikel aufweist.

30. Gerät nach Anspruch 29, **dadurch gekennzeichnet, daß** die Partikel Glaspartikel, Kalziumphosphatpartikel, Keramikpartikel, insbesondere Al₂O₃-Partikel, Hydroxylapatitpartikel, Fluorapatitpartikel, Kalziumfluoridpartikel, Kunststoffpartikel, Kompositpartikel, Salzpartikel, Carbonatpartikel, Flußspatpartikel usw. oder Mischungen der vorgenannten Partikel umfassen.

31. Gerät nach Anspruch 30, **dadurch gekennzeichnet, daß** die mittlere Korngröße der Partikel weniger als 50 µm, vorzugsweise weniger als 20 µm, nochmals vorzugsweise unter 10 µm bzw. 5 µm und besonders bevorzugt zwischen etwa 0,5 und etwa 2 µm beträgt.

32. Gerät nach einem der Ansprüche 1-31, **dadurch gekennzeichnet, daß** das Diagnosefluid mindestens ein Viskositäts-Einstellmittel enthält.

33. Gerät nach einem der Ansprüche 1-32, **dadurch gekennzeichnet, daß** das Diagnosefluid mindestens eine organische Gewebereste auflösende Substanz oder eine Gewebe ätzende Substanz enthält.

34. Gerät nach einem der Ansprüche 1-33, **dadurch gekennzeichnet, daß** das Diagnosefluid mindestens eine Behandlungssubstanz enthält.

## Claims

1. Apparatus for determining the remineralization ability of hard tissue, in particular carious dental tissue, comprising a measuring device (46, 60, 86, 88) for local measurement of at least one physical property of the hard tissue cooperating with a measuring head (40) having a rod-shaped probe section (42) adapted to be inserted into a working passage (42) formed in the hard tissue and having a sealing element (54) cooperating with a portion of the surface of the tissue, **characterized in that** a measuring element (60; 66) determining the porosity of the hard tissue communicates with the probe section (42) and **in that** the measuring head communicates with a diagnostic fluid source (76; 80) being under a pressure being different from normal pressure and **in that** the measuring device is formed such that it measures the leakage of diagnostic fluid through the tissue to be diagnosed.

2. The apparatus as in claim 1, **characterized by** a position sensor (48-52) being responsive to the axial position of the probe section (42) with respect to one of the surfaces of the tissue.

3. The apparatus as in claim 2, **characterized in that** the probe position sensor (48-52) is formed with an input member (48) cooperating with the surface of the tissue which is arranged on the probe section (42) for axial movement and is supported from a stationary portion of the apparatus by spring means (50) which cooperates with a force or deformation sensor (52).

4. The apparatus as in claim 3, **characterized by** an evaluation unit (88) receiving the output signal of the probe position sensor (48-52) and the output signal of the measuring element, and which compares the output signals of the latter received for different positions of the probe section (42) with one another, to which end the measuring signals obtained for different positions of the probe are memorized as a function of the output signal of the probe position sensor (48-52).

5. The apparatus as in one of claims 1 to 4, wherein the measuring element has a measuring axis being radial with respect to the axis of the probe section (42), **characterized in that** the probe section (42) is rotatably (188-122) arranged on the measuring head (40).

6. The apparatus as in one of claims 1 to 5, **characterized in that** the measuring head (40) is connected to a grip member (36) preferably being rotatable with respect to the longitudinal axis thereof.

7. The apparatus as in claim 6, **characterized in that** the longitudinal axis of the grip member (36) and the axis of the measuring head (40) are inclined with respect to one another preferably forming an angle between about 30° and about 90°, preferably of about 60°.

8. The apparatus as in claim 6 or 7, **characterized in that** the grip member (36) is connected to a supply cable (101) via a rotary cuppling (99).

9. The apparatus as in one of claims 1 to 8, **characterized in that** control means (94) are provided to define the flow of diagnostic fluid supplied.

10. The apparatus as in claim 9, **characterized in that** the control means comprise input means (94) carried by the grip members (36).

11. The apparatus as in claim 9 or 10, **characterized in that** the control means comprise numerical control means (88).

12. The apparatus as in claim 11, **characterized in that** the numerical control means operates such that the supply of diagnostic fluid is controlled by such activation that a predetermined output signal is obtained at the measuring element (60).

13. The apparatus as in one of claims 1 to 12, **characterized in that** the probe section (42) is of cylindrical shape.

14. The apparatus as in one of claims 1 to 13, **characterized in that** the fluid is a gas and **in that** the measuring head (40) is connectable to the fluid source (76; 80) by means of a switch valve (62) having a closed position and **in that** a pressure sensor (60) is connected to the probe space which is defined by the measuring head (40) and the tissue.

15. The apparatus as in claim 14, **characterized in that** the measuring head (40) comprises a pressure accumulator communicating with the probe space.

16. The apparatus as in one of claims 1 to 13, **characterized in that** the diagnostic fluid is a liquid and **in that** a flow meter (66) is provided in a supply line extending from the fluid source (76) to the measuring head (40).

17. The apparatus as in claim 16, **characterized in that** the flow meter (66) comprises a capillary pipe and means for supplying bubbles of gas into the stream up end of the capillary pipe.

18. The apparatus as in one of claims 1 to 17 in combination with claim 3, **characterized in that** the sealing element (54) is carried by the free end face of the input member (48) of the probe position sensor (48-52).

19. The apparatus as in one of claims 1 to 17, **characterized in that** the probe section (46) is a cylindrical pipe the end of which is closed (44) and the circumferential wall of which is formed with at least one fluid opening.

20. The apparatus as in claim 19, **characterized in that** the probe section (42) has at the end thereof being remote from the free end a radially flaring preferably conical shoulder (274) and **in that** a sealing tube (276) is arranged on the outer surface of the probe section (42), the end of the sealing tube being remote from the free end of the probe section (42) being adapted to be moved onto the sealing shoulder (274) under expansion thereof.

21. The apparatus as in one of claims 1 to 20, **characterized in that** the measuring head (40) is alternatingly connectable to a positive pressure fluid source (76) and a negative pressure fluid source (80) by means of a switch valve (72).

22. The apparatus as in claim 21 in combination with claim 16, **characterized by** means (102) for recovering the volume of liquid sucked from the measuring head (40).

23. The apparatus as in one of claims 1 to 22, **characterized in that** the fluid source (76; 80) for diagnostic fluid is a positive pressure source providing diagnostic fluid having a positive pressure of 0,1 to 0,8 bar, preferably 0,1 to 0,5 bar.

24. The apparatus as in one of claims 1 to 23, **characterized by** means for exposing the diagnostic fluid to sound or ultrasound.

25. The apparatus as in claim 24, **characterized by** a feed member (42b) transporting the diagnostic fluid and being exposed to sound or ultrasound, the feed member being insertable into the probe channel (32).

26. The apparatus as in claim 25, **characterized in that** the supply member (42b) is hollow **in that** the interior of the supply member (42b) is connectable to a negative pressure source.

27. The apparatus as in one of claims 1 to 26, **characterized in that** the diagnostic fluid contains a marker substance being selectively responsive to carious tissue.

28. The apparatus as in claim 27, **characterized in that** the marker substance is a colorant like Fuchsin, an X-ray contrast material, a radio nuclide or an electrolyte.

29. The apparatus as in one of claims 1 to 28, **characterized in that** the diagnostic fluid comprises inert particles.

30. The apparatus as in claim 29, **characterized in that** the particles comprise glass particles, potassium phosphate particles, ceramic particles, particularly Al₂O₃-particles, hydroxyapatite particles, fluoroapatite particles, potassiumfluoride particles, plastics particles, composite particles, salt particles, carbonate particles, fluorspar-particles or mixtures of the precited particles.

31. The apparatus as in claim 30, **characterized in that** the average drain size of the particles is less that 50 µm, preferably less than 20 µm, again preferable less than 10 µm or 5 µm and particularly preferred between about 0,5 and about 2 µm.

32. The apparatus as in one of claims 1 to 31, **characterized in that** the diagnostic fluid includes at least one viscosity adjusting substance.

33. The apparatus as in one of claims 1 to 32, **characterized in that** the diagnostic fluid comprises at least one substance dissolving rests of organic tissue or a substance edging tissue.

34. The apparatus as in one of claims 1 to 33, **characterized in that** the diagnostic fluid comprises at least one treatment substance.

## Revendications

1. Appareil pour déterminer l'aptitude à la reminéralisation d'un tissu dur, en particulier d'un tissu dentaire carié, comportant un dispositif de mesure (46, 60, 86, 88) destiné à la mesure locale d'une propriété physique du tissu dur, et qui collabore avec une tête de mesure (40), laquelle présente un segment de sonde (42) en forme de bâton pouvant être inséré dans un canal de travail (32) dans le tissu dur, et présente un élément d'étanchéité (54) qui collabore avec un segment de la surface du tissu, **caractérisé en ce qu'**un élément de mesure (60 ; 66) qui détecte la porosité du tissu dur se trouve en relation avec le segment de sonde (42), et **en ce que** la tête de mesure (40) est en relation avec une source de fluide de diagnostic (76 ; 80) qui se trouve sous une pression différente de la pression normale, et le dispositif de mesure est élaboré pour mesurer la fuite du fluide de diagnostic par le tissu à examiner.

2. Appareil selon la revendication 1, **caractérisé par** un transmetteur de position (48-52) pour la position axiale du segment de sonde (42) par rapport à une surface limite du tissu.

3. Appareil selon la revendication 2, **caractérisé en ce que** le transmetteur de position de sonde (48-52) présente un élément d'entrée (48) collaborant avec la surface du tissu, lequel élément est disposé sur le segment de sonde (42) de façon à pouvoir être déplacé dans la direction axiale et repose par le biais d'un ressort (50) contre un segment d'appareil fixe auquel est coordonné un capteur de force ou de déformation (52).

4. Appareil selon la revendication 3, **caractérisé par** une unité d'évaluation (88) qui est alimentée par le signal de sortie du transmetteur de position de sonde (48-52) et par le signal de sortie de l'élément de mesure, et compare entre eux les signaux de sortie de ce dernier qui sont obtenus pour des positions différentes du segment de sonde (42), les signaux de mesure obtenus pour les différentes positions de sonde respectivement en fonction du signal de sortie du transmetteur de position de sonde (48-52) étant pour ce faire mémorisés.

5. Appareil selon l'une des revendications 1 à 4, dans lequel l'élément de mesure présente un axe de mesure radial par rapport à l'axe du segment de sonde (42), **caractérisé en ce que** le segment de mesure (42) est disposé sur la tête de mesure (40) de façon rotative (118-122).

6. Appareil selon l'une des revendications 1 à 5, **caractérisé en ce que** la tête de mesure (40) est montée sur un élément d'une poignée (36) et peut tourner de préférence autour de son axe longitudinal.

7. Appareil selon la revendication 6, **caractérisé en ce que** l'axe longitudinal de l'élément de poignée (36) et l'axe de la tête de mesure (40) sont inclinés l'un par rapport à l'autre et forment de préférence un angle compris entre environ 30° et environ 90°, de préférence encore d'environ 60°.

8. Appareil selon la revendication 6 ou 7, **caractérisé en ce que** l'élément de poignée (36) est reliée à un câble d'alimentation (101) par un raccord rotatif (99).

9. Appareil selon l'une des revendications 1 à 8, **caractérisé en ce que** des moyens (94) de réglage de la force d'alimentation en fluide de diagnostic sont prévus.

10. Appareil selon la revendication 9, **caractérisé en ce que** les moyens de réglage comprennent un organe d'entrée (94) porté par l'élément de poignée (36).

11. Appareil selon la revendication 9 ou 10, **caractérisé en ce que** les moyens de réglage comprennent une commande par programme (88).

12. Appareil selon la revendication 11, **caractérisé en ce que** la commande par programme travaille de façon telle que l'apport de fluide de diagnostic est commandé par excitation de façon telle que l'on obtient un signal de sortie prédéfini au niveau de l'élément de mesure (60).

13. Appareil selon l'une des revendications 1 à 12, **caractérisé en ce que** le segment de sonde (42) est cylindrique.

14. Appareil selon l'une des revendications 1 à 13, **caractérisé en ce que** le fluide est un gaz et **en ce que** la tête de mesure (40) peut être reliée à la source de fluide (76 ; 80) via une soupape de commande (62) qui présente une position de fermeture, et **en ce qu'**un manomètre (60) est raccordé à l'espace de mesure, qui est délimité par la tête de mesure (40) et le tissu.

15. Appareil selon la revendication 14, **caractérisé en ce que** la tête de mesure (40) présente un accumulateur de pression (58) relié à l'espace de mesure.

16. Appareil selon l'une des revendications 1 à 13, **caractérisé en ce que** le fluide de diagnostic est un liquide, et **en ce qu'**un débitmètre (66) est disposé dans la conduite d'alimentation allant de la source de fluide (76) à la tête de mesure (40).

17. Appareil selon la revendication 16, **caractérisé en ce que** le débitmètre (66) présente un tube capillaire et des moyens permettant d'injecter des bulles de gaz au niveau de l'extrémité aval du tube capillaire.

18. Appareil selon l'une des revendications 1 à 17 en relation avec la revendication 3, **caractérisé en ce que** l'élément d'étanchéité (54) est porté par la face avant libre de l'élément d'entrée (48) du transmetteur de position de sonde (48-52).

19. Appareil selon l'une des revendications 1 à 17, **caractérisé en ce que** le segment de sonde (42) est un tube cylindrique fermé à l'extrémité (44), qui présente dans sa paroi périphérique au moins une ouverture de passage de fluide.

20. Appareil selon la revendication 19, **caractérisé en ce que** le segment de sonde (42) présente au niveau de son extrémité éloignée de l'extrémité libre un épaulement (274) qui s'élargit dans la direction radiale, de préférence en forme de cône, et **en ce qu'**un tuyau d'étanchéité (276) est disposé sur le côté externe du segment de sonde (42) et peut être poussé, par son extrémité éloignée de l'extrémité libre du segment de sonde (42), sur l'épaulement d'étanchéité (274) en s'élargissant.

21. Appareil selon l'une des revendications 1 à 20, **caractérisé en ce que** la tête de mesure (40) peut être reliée par une soupape d'inversion (72) en alternance avec une source de fluide en surpression (76) et une source de fluide en dépression (80).

22. Appareil selon la revendication 21 en relation avec la revendication 16, **caractérisé par** des moyens (102) permettant de capter le volume de fluide aspiré de la tête de mesure (40).

23. Appareil selon l'une des revendications 1 à 22, **caractérisé en ce que** la source de fluide de diagnostic (76 ; 80) est une source de surpression qui met à disposition du fluide de diagnostic présentant une surpression de 0,1 à 0,8 bar, de préférence de 0,1 à 0,5 bar.

24. Appareil selon l'une des revendications 1 à 23, **caractérisé par** des moyens permettant d'alimenter le fluide de diagnostic en sons ou en ultrasons.

25. Appareil selon la revendication 24, **caractérisé par** un élément d'alimentation (42b), chargé en sons ou ultrasons, pour le fluide de diagnostic, qui peut être introduit dans un canal de travail (32).

26. Appareil selon la revendication 25, **caractérisé en ce que** l'élément d'alimentation (42b) est creux et **en ce que** son espace interne peut être relié à une source de dépression (80).

27. Appareil selon l'une des revendications 1 à 26, **caractérisé en ce que** le fluide de diagnostic contient une substance de marquage qui réagit de façon sélective à du tissu carié.

28. Appareil selon la revendication 27, **caractérisé en ce que** la substance de marquage comprend un colorant comme la fuchsine, un produit de contraste radiologique, un radionucléide ou un électrolyte.

29. Appareil selon l'une des revendications 1 à 28, **caractérisé en ce que** le fluide de diagnostic présente des particules inertes.

30. Appareil selon la revendication 29, **caractérisé en ce que** les particules sont des particules de verre, des particules de phosphate de calcium, des particules céramiques, en particulier des particules d'Al₂O₃, des particules d'hydroxylapatite, des particules de fluorapatite, des particules de fluorure de calcium, des particules synthétiques, des particules composites, des particules de sel, des particules de carbonate, des particules de fluorine, etc., ou des mélanges des particules précitées.

31. Appareil selon la revendication 30, **caractérisé en ce que** la taille moyenne des particules est inférieure à 50 µm, de préférence inférieure à 20 µm, de préférence encore inférieure à 10 µm ou 5 µm, et de façon particulièrement préférée comprise entre environ 0,5 et environ 2 µm.

32. Appareil selon l'une des revendications 1 à 31, **caractérisé en ce que** le fluide de diagnostic contient au moins un agent d'ajustement de la viscosité.

33. Appareil selon l'une des revendications 1 à 32, **caractérisé en ce que** le fluide de diagnostic contient au moins une substance qui dissout les tissus organiques résiduels ou une substance qui attaque les tissus.

34. Appareil selon l'une des revendications 1 à 33, **caractérisé en ce que** le fluide de diagnostic contient au moins une substance de traitement.
